# EUROPEAN PATENT APPLICATION

(11) **EP 4 027 140 A1**
(43) Date of publication of application: **13.07.2022**
(21) Application number: 22150299.0
(22) Date of filing: 05.01.2022
(51) Int. Cl.: G01N 33/493, G01N 27/333

(54) **ANALYSIS DEVICE FOR UNTREATED URINE**

(30) Priority: 11.01.2021 IT 202100000341
(71) Applicant: Redoak S.r.l., 20122 Milano (IT)
(72) Inventor: ORSENIGO, Renzo, MILANO (IT); MANZONI, Angelo, MILANO (IT); MANZONI, Giulia, MILANO (IT)
(74) Representative: Lunati & Mazzoni S.r.L.

(57) **Abstract**

It is provided an urine analysis device (1) for analysing an untreated urine sample and determining the physio-chemical characteristics of the urine sample; the analysis device (1) comprising a sampler inlet (2) adapted to be put in a fluid passage connection with a patient and to dose a portion of fluid to be analysed; and an analysis station (3) placed in fluid passage connection with the sampler inlet (2), suitable for carrying out the analysis of at least part of the portion of fluid to be analysed and comprising a plurality of ion-selective electrodes (30) including ion-selective electrode K⁺ (31) for potassium ions (K⁺) comprising a support body (310), a first internal reference electrode (311), a reference solution (312) and a selective element (313) comprising a substrate (3133), and a membrane (3130) comprising at least one silicone polymer (3131) and an ionophore polymer ( 3132) and an ion-selective electrode NH4⁺ (32), and in which the analysis station (3) is configured to correct the measurements of the ion-selective electrode NH4 + (32) by means of a predetermined correction algorithm in such a way as to replicate the accuracy of the ion-selective electrode K⁺ (31) on the ion-selective electrode NH4⁺ (32) exclusively by computational calculations.

## Description

The present invention relates to an analysis device of urine suitable for analysing a sample of untreated urine of the type specified in the preamble of the first claim. Specifically, the device is adapted to determine the physio-chemical characteristics value of said urine sample and in particular comprises at least one ion-selective electrode for potassium ions.

In addition, the device comprises at least one ion-selective electrode for ammonium ions and an electrode for determining the pH, the simultaneous presence of which in the device allows to have indications relating to the metabolic state of renal activity and in particular metabolic acidosis.

Finally, the present invention relates to a method for manufacturing an ion-selective electrode for potassium ions suitable for use in an analysis device for untreated urine.

Urine analysis devices are currently known, specifically devices for the electrochemical analysis of urine, as urinalysis is one of the main methods of analysis to demonstrate or exclude many pathologies/problems such as structural lesions, the presence of infectious agents, an alteration of one or more organs and more.

In detail, some of the components or ions most interested by urinalysis devices, due to its importance in the human body, are the potassium ion K⁺ and the ammonium ion NH4⁺.

In fact, the quantity of ammonium ion NH4⁺ allows to evaluate, in emergency conditions and/or resuscitation in intensive care, the ability of the kidney to maintain the correct acid-base balance and the quantity of potassium ion K⁺ allows to evaluate alteration of functionality. of neuromuscular and cardiac tissue.

The known art provides for the use of ion-selective electrodes, sensors capable of indirectly measuring, therefore in contact with a solution, usually diluted urine, the concentration of a specific ion present in said solution. The electrode is commonly equipped with a sensitive part (membrane), which generates a potential difference, due to the different concentration between the reference solution and the one containing the analyte. This potential difference is converted into ion concentration, through mathematical equations which are determined with respect to an arbitrary reference system (calibration).

The formulations of said selective membranes for potassium ions are normally based on polyvinyl chloride (PVC) matrices plasticised with hydrophobic compounds such as Bis (2-ethyl hexyl) phtalate, 2 -Nitrophenyl octyl ether, Bis (2 - Ethyl hexyl) sebacate, Dibutyl phthalate and additives such as Potassiumtetrakis para chloro phenyl borate.

The plasticizers offer appropriate ion mobility on the polymer surface and the resulting electrical resistance of the membrane reduces noise in analyses and increases selectivity.

The known technique described comprises some important drawbacks.

In particular, the membranes for the analysis of potassium ions normally used for ion-selective electrodes that are used on human blood analysers are not suitable for determining the same components in undiluted urine and, in order to do so, require a dilution ranging from a ratio of 1: 3 up to a ratio of 1:10. This is evident by comparing the analytical results with reference instrumentation such as flame photometry or with ISE instrumentation, i.e. instrumentation that uses Ion-Selective Electrodes, which dilutes the sample with known solutions in order to perform the analysis (indirect potentiometry).

Furthermore, the chemical structure, the distribution of the ionic charges and the composition of said membranes are not suitable for obtaining accurate and repeatable analytical results when in contact with undiluted urine.

For example, the use of PVC-based matrices, intended as matrices in which there is the dissolution of high molecular weight PVC powder in the matrix of the sensitive membrane and where PVC therefore represents a specific component, is not suitable for analysing continuous concentration of K⁺ in undiluted and/or untreated urine during the monitoring of ICU patients with impaired cardiac or renal function. Furthermore, the use of PVC-based matrices is not suitable for analysing the concentration of the K⁺ ion in an extended range of possible variation and the electrolyte balance forces medical personnel to delay the clinical evaluation. Furthermore, the electrodes of the known art for the manufacture of the membrane of the same electrode, and more specifically for the connection of the sensitive membrane to the substrate, use adhesives which can invalidate the measurement of the specific ion.

Finally, the use of corona ethers, as an alternative to ionophores, provides satisfactory results with blood and serum but presents the previously described problems of accuracy and repeatability with undiluted urine.

In this situation, the technical task underlying the present invention is to devise an untreated urinalysis device capable of substantially obviating at least part of the aforementioned drawbacks.

Within the scope of said technical task, it is an important object of the invention to obtain a device suitable for determining the value of physicochemical characteristics of an untreated urine sample and in particular comprises at least one ion-selective electrode for potassium ions.

Furthermore, an important object of the present invention is to provide an ion-selective electrode for K⁺ ions which provides a reliable and valid result from the diagnostic point of view in emergency conditions.

Another important object of the invention is to provide an ion-selective electrode for K⁺ ions capable of being put in direct contact with undiluted urine and providing reliable results.

Furthermore, an important object of the present invention is a method for the manufacture of an ion-selective electrode for potassium ions which does not use adhesives for the connection of the sensitive membrane to the substrate. Furthermore, an important object of the present invention is to provide an untreated urinalysis device comprising an ion-selective electrode for NH4⁺ ions capable of determining the concentration of the ammonium ion in an accurate way through the contribution of the analysis accuracy of the ion-selective electrode for K⁺ ions. Finally, an important object of the present invention is a method for manufacturing an ion-selective electrode such as to make accurate and simultaneous the measurement of the ammonium ion by potentiometric way in the undiluted urine sample.

The technical task and the specified aims are achieved by an untreated urinalysis device comprising at least one ion-selective electrode for potassium ions as claimed in the attached claim 1.

Preferred technical solutions are highlighted in the dependent claims.

The characteristics and advantages of the invention are clarified below by the detailed description of preferred embodiments of the invention, with reference to the accompanying drawings, in which:
the **Fig. 1** analysis device according to the present invention;
the **Fig. 2** ion-selective electrode for potassium ions;
the **Fig. 3** shows a first graph of results obtained with the analysis device according to the present invention; and
the **Fig. 4** shows a second graph of results obtained with the analysis device according to the present invention
the **Fig. 5** shows a graph representing a straight line obtained by plotting the concentration of the potassium ion (K⁺) and on the ordinate the concentration, with unit of measurement mM, of the ammonium ion (NH₄⁺) read by the relative electrodes; and
the **Fig. 6** shows a graph representing a straight line obtained by plotting in the abscissa the concentration, with unit of measurement mM, read of the ammonium ion (NH+) by the relative electrode and in the ordinate the difference, with unit of measurement mM, between this reading and the corresponding measurement obtained with the spectrophotometer (DNH₄).

In the present document, the measurements, values, shapes and geometric references (such as perpendicularity and parallelism), when associated with words like "about" or other similar terms such as "approximately" or "substantially", are to be considered as except for measurement errors or inaccuracies due to production and/or manufacturing errors, and, above all, except for a slight divergence from the value, measurements, shape, or geometric reference with which it is associated. For instance, these terms, if associated with a value, preferably indicate a divergence of not more than 10% of the value.

Moreover, when used, terms such as "first", "second", "higher", "lower", "main" and "secondary" do not necessarily identify an order, a priority of relationship or a relative position, but can simply be used to clearly distinguish between their different components.

Unless otherwise specified, as results in the following discussions, terms such as "treatment", "computing", "determination", "calculation", or similar, refer to the action and/or processes of a computer or similar electronic calculation device that manipulates and/or transforms data represented as physical, such as electronic quantities of registers of a computer system and/or memories in, other data similarly represented as physical quantities within computer systems, registers or other storage, transmission or information displaying devices.

The measurements and data reported in this text are to be considered, unless otherwise indicated, as carried out in the ICAO International Standard Atmosphere (ISO 2533: 1975).

With reference to the Figures, the analysis device of urine according to the invention is globally indicated with the number **1.**

Specifically, the analysis device 1 is adapted to analyse a sample of untreated urine and to determine the value of the physio-chemical characteristics of the urine sample. As will be clear, in this document we mean by untreated urine sample urine as it is taken from the patient, therefore urine that has not undergone active changes in temperature, dilutions, oxidations, i.e. urine without the addition of reagents and/or solvents of any kind.

The analysis device 1 comprises at least one sampler inlet **2** adapted to be put in a fluid passage connection with the body fluids, in particular urine, emitted by a patient and to dose a portion of fluid to be analysed, or rather a sample of untreated urine. Preferably, the device 1 further comprises an analysis station **3** placed in a fluid passage connection with the sampler inlet 2 and suitable for carrying out an analysis of at least part of the portion of fluid to be analysed.

The device 1 described up to now is, for example, similar to a urinalysis device as described in patent application EP2510877A1 owned by the applicant of the present patent application.

Again, preferably, the analysis station 3 comprises a plurality of ion-selective electrodes **30.**

In the present document the term ion-selective electrode 30 is used to indicate an entire electrochemical cell capable of carrying out an electrochemical analysis of the fluid and determining at least one parameter, for example a potential difference, for each of the physicochemical characteristics. In the case of urinalysis, the analysis station 3 can comprise a plurality of electrodes 30 suitable for analysing, for example, some of the following characteristics of the fluid: pH, contents of sodium, potassium, ammonium, chloride and more.

Preferably, according to the present invention, the analysis station 3 comprises at least one ion-selective electrode K⁺ **31** for potassium ions K⁺.

Again, preferably, according to the present invention, the analysis station 3 comprises at least one ion-selective electrode K⁺ **31** for potassium K + ions and one ion-selective electrode NH4⁺ **32** for ammonium ion NH4⁺. The ion-selective electrode NH4⁺ 32 for ammonium ion NH4⁺ is, for example, an electrode known to those skilled in the art.

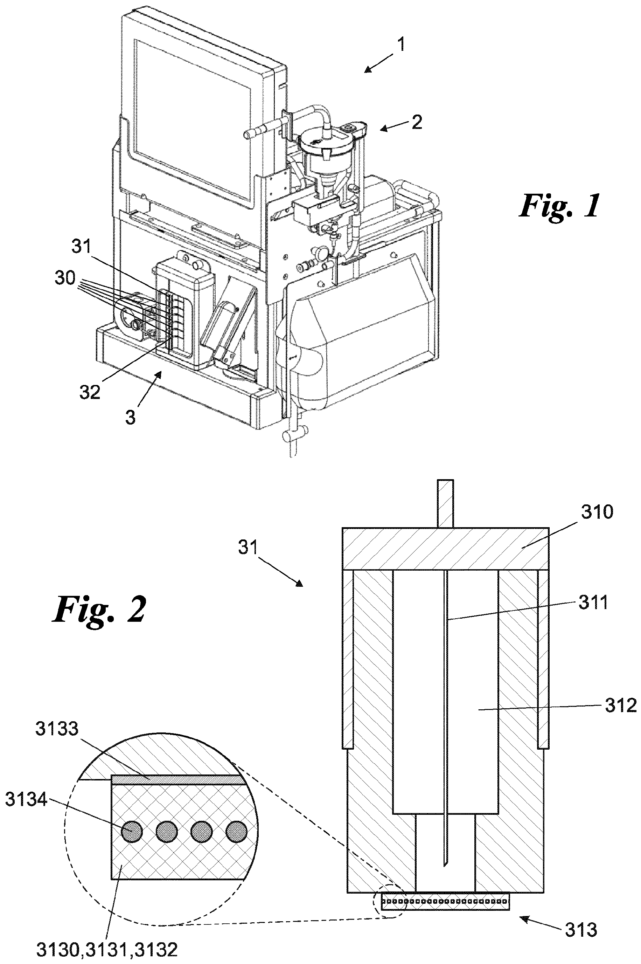

Preferably, as shown in Fig. 2, the ion-selective electrode K⁺ 31 for potassium ions K⁺ comprises a support body **310**, a first internal reference electrode **311**, a reference solution **312** and a selective element **313.** Preferably at least the selective element 313 is adapted to be in direct contact with a fluid bed of urine. Said selective element 313 is the part of the ion-selective electrode K⁺ 31 whose potential varies as a function of the concentration of a given ionic species.

The structure of the electrode 31 is broadly similar to the structure of any electrochemical cell of the known art.

Preferably, the selective element 313 comprises a membrane **3130** and a substrate **3133.** The membrane 3130 is in fact the sensitive element capable of being placed at least partially in direct contact with the fluid to be analysed, that is, undiluted urine. The membrane 3130 comprises a silicone polymer **3131** and an ionophore **3132.** It also comprises, internally, a structural support **3134,** such as a stiffening network, for example polymeric, or other. The structural support 3134 is able to give greater mechanical compactness to the membrane 3130.

In detail, the membrane 3130, preferably including the structural support 3134, for its realization, is initially preferably placed on a support made of polymeric material and in particular of PTFE. The same membrane 3130 3130 with the structural support 3134 is then mechanically detached from the PTFE support and cut, preferably by punching and placed without gluing on the substrate 3133, preferably consisting of a PVC support preferably having the same diameter.

Preferably, according to the present invention, the silicone polymer 3131 is polydimethylsiloxane or PDMS. Even more preferably, the silicone polymer 3131 is room temperature curing polydimethylsiloxane also called PDMS RTV.

Again, the ionophore 3132 is preferably valinomycin. In one embodiment of the present invention, preferably, the substrate 3133 is a rigid polyvinyl chloride or PVC substrate.

In more preferably, according to the present invention, the membrane 3130 comprises a percentage by weight of silicone polymer 3131 comprised between 97.5% and 98%. Again, preferably, the membrane 3130 comprises a percentage by weight of ionophore 3132 comprised between 2% and 2.5%. Preferably, the reference solution 312 is a 50 millimolar solution of KCI.

Finally, preferably, the membrane 3130 has a thickness comprised between 250 µm and 300 µm.

As anticipated, the present invention also relates to a method **10** for manufacturing a selective element 313 such as that just described in the document.

Preferably, therefore, the selective element 313 is part of an ion-selective electrode K⁺ 31 for potassium ions K⁺, this selective element 313 adapted to be positioned in direct contact with a bed of undiluted urine.

The method 10 according to the present invention preferably comprises a step consisting in preparing a compound comprising at least one silicone polymer 3131, an ionophore 3132 and a volatile solvent. In a preferred embodiment the volatile solvent is for example dichloromethane.

Again, method 10, in the following, provides for mixing the compound, preferably mildly and preferably for 15 minutes.

In addition, the method 10 preferably comprises a step consisting in pouring the compound onto a support made of polymeric material and in particular of PTFE. Subsequently, method 10 preferably comprises a step in which the compound is allowed to solidify on the support, preferably for at least 24 hours and preferably at room temperature until a membrane 3130 is formed.

In this step, the structural support 3134 can also be inserted inside of the membrane 3130.

Finally, the method 10 according to the present invention provides to mechanically separate the membrane 3130 from the PTFE support and then to punch said membrane 3130. by means of a punch, and to deposit it on a substrate 3133, preferably in PVC, to forming the selective element 313.

The selective element which is then coupled to the remaining part of the ion-selective electrode K⁺ 31 and inserted in the device 1.

The device 1 according to the invention achieves important advantages.

In fact, advantageously, the ion-selective silicone matrix membranes allow to obtain a linearity range extended from 10⁻³ M to 10⁻¹ M on undiluted urine. Advantageously, the silicone matrix adopted for the electrode just described allows to obtain accurate potassium values in a wide concentration range between 1 mM and 150 mM. The reading accuracy and reproducibility of this membrane allow to obtain, through specific correction algorithms, correct measurements of the ammonium concentration (NH4⁺) in the urine as such.

For example, the correction algorithm can be traced back to an equation such as: NH4 _{corr} = (NH4 _{read} ⁺/ - A * K⁺ _{read} ⁺/ - B) / C; where A, B and C are constants deriving from the comparison with reference methods for the NH4⁺ (i.e. colorimetric analysis on Cobas Integra ^{®} 400 - Roche, analyses based on ion chromatography and spectrophotometry).

Preferably, the constant A of the equation in question represents the slope of the straight line obtained by reporting the concentration of the potassium ion (K⁺) on the abscissa and the concentration of the ammonium ion (NH₄⁺) read by the relative electrodes on the abscissa.

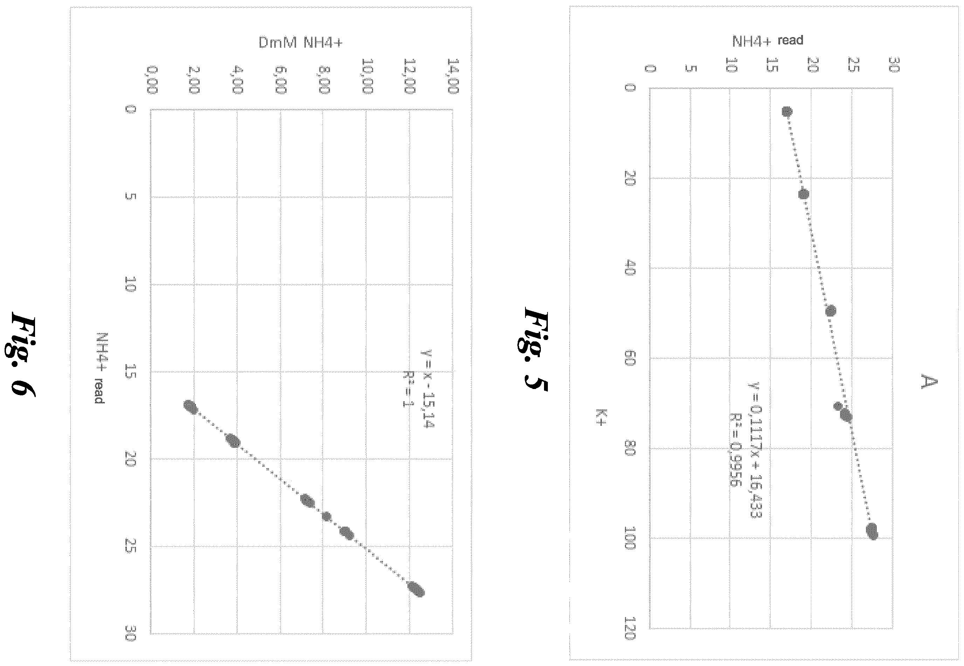

In the example shown in Fig. 5, the constant A is equal to 0.1117 and substantially represents the influence that the potassium ion (K⁺) has on the concentration of the ammonium ion (NH₄⁺).

The constant B represents the algebraic sum between the intercept of the straight line obtained by placing the concentration of the ammonium ion (NH₄⁺) read by the relative electrode on the abscissa and the difference between the latter and the corresponding spectrophotometer measurement on the abscissa (dNH₄) and the intercept with the Y axis obtained from the graph relating to constant A.

In the example represented graphically by figures 5 and 6, the constant B is obtained from the algebraic sum of the two intercepts (16.433-15.14 = 1.293).

Starting from the equation 0.117 * K_{read} + 1.293 we obtain the correction factor to be applied to the reading of the ammonium ion (NH₄⁺) and therefore the corrected NH4⁺ is given by NH₄⁺_{read} - (A * K_{read} + B).

The constant C is preferably obtained by correlating all the results obtained from the various solutions analysed, that is, from the experimental data.

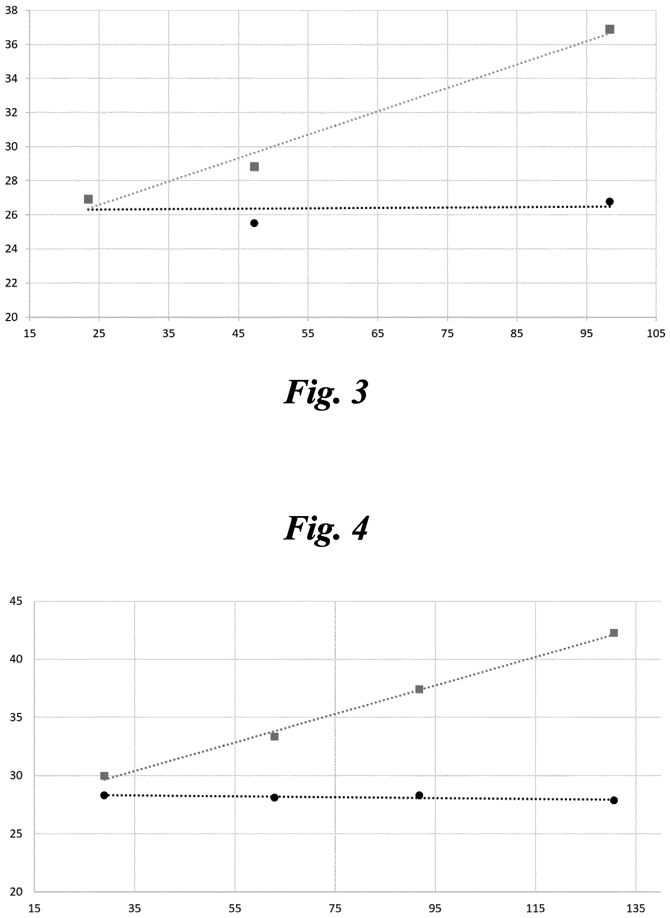

The graphs in Fig. 3 and Fig. 4 respectively show two measurements of the NH4⁺ (at fixed concentration), where the values (in mM) of the quantity of the K⁺ are present in the abscissa, while the values are present in the ordinate (in mM) of the NH4⁺. The graphs show, in gray and with square points, the values of the NH4⁺, not corrected according to the reading of the K⁺, (a deviation of the concentration of the NH4⁺ ion is observed in growth with increasing concentration of the ion K⁺), while, in black and with circular dots, the values of the NH4⁺ corrected for the reading of the K⁺ (the disappearance of the concentration deviation of the NH4⁺ ion is observed as the concentration of the K⁺ ion increases). It is possible to check, in the same graphs, the accuracy of the correct measurements.

In fact, advantageously, thanks to the simultaneous presence of an ion-selective electrode for ammonium ions and of the innovative ion-selective electrode for potassium ions in the device according to the present invention, a correct measurement of the K⁺ ion when analysing an untreated urine sample allows an accurate correction through the correction algorithm identified above.

In other words, the analysis station 3 is configured to correct the measurements of said ion-selective electrode NH4⁺ 32 by means of a predetermined correction algorithm in such a way as to replicate the precision of the ion-selective electrode K⁺ 31 on the ion-selective electrode NH4⁺ 32 exclusively through computational calculations, that is without the need for particular structural measures, for example as the selective element 313, to obtain precise and accurate results.

Basically, the ion-selective electrode NH4⁺ 32 in its measurements is functionally influenced by the ion-selective electrode K⁺ 31 and, therefore, by the presence of the K⁺ ion which is added algebraically to that of the NH4⁺ ion present in the sample under analysis.

The ion-selective electrode K⁺ 31 is able to determine the relative K⁺ ion in an accurate way, since it is not influenced by interferents present in the urine matrix, and to allow the use of a known correction to be applied to the measurement made by the ion-selective electrode NH4⁺ 32 and such as to bring the same to accurate values that are superimposable to those of an instrument taken in comparison. Therefore, the device 1 allows to determine simultaneously and with accuracy, through ion-selective electrodes K⁺ 31 and NH4⁺ 32 and on urine as such, the concentration of ammonium and potassium ions.

The determination of the K⁺ ion and the NH4⁺ ion takes place on the same analyser, on the same analysis matrix, in the same time interval and therefore continuously, or simultaneously, with respect to the initial time of the measurement itself.

The method 10 according to the present invention, thanks to the casting or production of the silicone matrix K⁺ on a rigid support that creates the seal in the absence of gluing: eliminating pollution of the selective membrane with foreign elements and modifying the concentration of the selective components at internal of the same with consequent extension of the useful life of the electrode. Advantageously again, the ion-selective electrode 31 for potassium ions K⁺, after at least 24 hours of conditioning in a 100 mM solution of KCI, shows a slope of 56mV/dec at 37 ° C and excellent stability in the presence of sodium concentrations ranging from 1 and 250 mM, ammonium cations between 1 and 150 mM and chlorine ions between 10 and 400 mM at pH between 3.5 and 8.5 and with a voltage deviation of 0.1 mV every 30 minutes.

Furthermore, the accuracy of the ion-selective electrode K+ for K + ions occurred in the concentration range 1 mM and 150 mM on untreated urine, in the presence of sodium ion concentrations between 1 and 250 mM, ammonium ions NH4⁺ between 1 and 150 mM, chloride ions CI- between 10 and 400 mM and pH between 3.5 and 8.5

A further advantage is given by the fact that the sensitive element 313 deposited and not glued on a rigid support 3133 eliminates transfer of pollutants and extraneous to the composition of the sensitive element 313.

All this has determined an increase in the useful life of the ion-selective electrode K⁺ 31 prepared with method 10 compared to those normally formulated with substances containing polymers, plasticizers, ionophores and additives and normally used on analysis instruments for blood and diluted urine.

Still, considering the materials now, advantageously according to the present invention, PVC does not enter the composition of the membrane because an RTV product is used in the preparation phase, and in the polymer formation phase (membrane still liquid) a cross-linking plane is used as a cross-linking plane. neutral substance such as PTFE. The invention is susceptible of variants falling within the scope of the inventive concept defined by the claims.

## Claims

1. Analysis device (1) of urine adapted to analyse a sample of untreated urine and to determine the value of the physio-chemical characteristics of said urine sample;
said analysis device (1) comprising a sampler inlet (2) adapted to be put in a fluid passage connection with a patient and to dose a portion of fluid to be analysed; and an analysis station (3) placed in fluid passage connection with said sampler inlet (2), suitable for carrying out said analysis of at least part of said portion of fluid to be analysed and comprising a plurality of ion-selective electrodes (30) including at least:
- an ion-selective electrode K⁺ (31) for potassium ions (K⁺) comprising a support body (310), a first internal reference electrode (311), a reference solution (312) and a selective element (313) comprising a substrate (3133) and a membrane (3130) including a silicone polymer (3131) and an ionophore polymer (3132), and
- an NH4⁺ ion-selective electrode (32)
said device (1) being **characterized by**
- said analysis station (3) is configured to correct the measurements of said NH4⁺ ion-selective electrode (32) by means of a predetermined correction algorithm in such a way as to replicate the precision of said ion-selective electrode K⁺ (31) on said NH4⁺ ion-selective electrode (32) exclusively through computational calculations.

2. Device (1) according to any one of the preceding claims, wherein said silicone polymer (3131) is polydimethylsiloxane.

3. Device (1) according to any one of the preceding claims, wherein said silicone polymer (3131) is polydimethylsiloxane cured at room temperature.

4. Device (1) according to any one of the preceding claims, wherein said ionophore (3132) is valinomycin

5. Device (1) according to any one of the preceding claims, wherein said membrane (3130) comprises a percentage by weight of said silicone polymer (3131) comprised between 97.5% and 98%.

6. Device (1) according to any one of the preceding claims, wherein said membrane (3130) comprises a percentage by weight of said ionophore (3132) comprised between 2% and 2.5%.

7. Device (1) according to any one of the preceding claims, wherein said substrate (3133) is a PVC structural support.

8. Device (1) according to any one of the preceding claims, wherein said membrane comprises, internally, a structural support (3134).

9. Device (1) according to any one of the preceding claims, wherein said membrane (3130) has a thickness of between 250 and 300 micrometres.

10. Method (10) for manufacturing of a said selective element (313) part of an ion-selective electrode K⁺ (31) for potassium ions (K⁺) according to any one of the preceding claims, wherein said method (10) comprises the steps of:
- preparing of a compound comprising at least one silicone polymer (3131), an ionophore (3132) and a volatile solvent,
- pouring the mixture onto a support,
- solidifying said compound on said support until a membrane (3130) is formed,
- punching said membrane (3130) and depositing said membrane on a substrate (3133) to form said selective element (313).

11. Method (1) according to claim 12, wherein said support is made of PTFE.

12. Method (1) according to claim 12 or 13, wherein a structural support (3134) is inserted in said membrane (3130) during solidification.

## Amended claims

### Amended claims in accordance with Rule 137(2) EPC.

1. Analysis device (1) of urine adapted to analyse a sample of untreated urine and to determine the value of the physio-chemical characteristics of said urine sample;
said analysis device (1) comprising a sampler inlet (2) adapted to be put in a fluid passage connection with a patient and to dose a portion of fluid to be analysed; and an analysis station (3) placed in fluid passage connection with said sampler inlet (2), suitable for carrying out said analysis of at least part of said portion of fluid to be analysed and comprising a plurality of ion-selective electrodes (30) including at least:
- an ion-selective electrode K⁺ (31) for potassium ions (K⁺) comprising a support body (310), a first internal reference electrode (311), a reference solution (312) and a selective element (313) comprising a substrate (3133) and a membrane (3130) including a silicone polymer (3131) and an ionophore polymer (3132), and
- an NH4⁺ ion-selective electrode (32)
said device (1) being **characterized by**
- said analysis station (3) is configured to correct the measurements of said NH4⁺ ion-selective electrode (32) by means of a predetermined correction algorithm in such a way as to replicate the precision of said ion-selective electrode K⁺ (31) on said NH4⁺ ion-selective electrode (32) exclusively through computational calculations,
- said silicone polymer (3131) is polydimethylsiloxane,
- said substrate (3133) is a PVC structural support,
- said ionophore (3132) is valinomycin.

2. Device (1) according to any one of the preceding claims, wherein said silicone polymer (3131) is polydimethylsiloxane cured at room temperature.

3. Device (1) according to any one of the preceding claims, wherein said membrane (3130) comprises a percentage by weight of said silicone polymer (3131) comprised between 97.5% and 98%.

4. Device (1) according to any one of the preceding claims, wherein said membrane (3130) comprises a percentage by weight of said ionophore (3132) comprised between 2% and 2.5%.

5. Device (1) according to any one of the preceding claims, wherein said membrane comprises, internally, a structural support (3134).

6. Device (1) according to any one of the preceding claims, wherein said membrane (3130) has a thickness of between 250 and 300 micrometres.

7. Device (1) according to any one of the preceding claims, wherein said correction algorithm is: NH4 _{corr} = (NH4 read +/- A * K+ read +/ - B) / C; where A, B C are constants.

8. Device (1) according to the preceding claims, wherein said A is equal to 0.1117, B is equal to 1.293 and C is 0.
